# EUROPEAN PATENT APPLICATION

(11) **EP 2 105 160 A1**
(43) Date of publication of application: **30.09.2009**
(21) Application number: 09000649.5
(22) Date of filing: 19.01.2009
(51) Int. Cl.: A61M 39/28

(54) **Roller clamp cover**

(30) Priority: 26.03.2008 JP 2008079818
(71) Applicant: Covidien AG, 8212 Neuhausen am Rheinfall (CH)
(72) Inventor: Kitani, Ichiro, Fukuroi Shizuoka 437-0004 (JP); Matsunaga, Michiyo, Fukuroi Shizuoka 437-0004 (JP)
(74) Representative: Olsson, Carl H.S.

(57) **Abstract**

To provide a roller clamp cover with which it is possible to maintain a set flow rate of a fluid flowing inside a medical tube to which a roller clamp is attached.

A roller clamp 10 for adjusting the flow rate of a fluid flowing inside a tube T is housed inside a roller clamp cover 20. The roller clamp cover 20 is then configured by a housing part 24 which can house the roller clamp 10 in a state in which the tube T projects from both sides, by rotation in reciprocating back and forth directions with a hinge linking part 23 at the centre, and positional offset prevention parts 25a, 25b etc. for preventing the roller clamp 10 from becoming positionally offset from the tube T when the roller clamp 10 is housed inside the housing part 24, by applying pressure contact to the outer peripheral surface of the tube T.

## Description

The present invention relates to a roller clamp cover which is attached to a roller clamp in order to adjust the flow rate of a medical fluid flowing inside a medical tube.

### [Prior Art]

Fluids such as drug solutions and blood are conventionally supplied to patients using a fluid transfusion or blood transfusion line comprising a medical tube. In these cases, a flow rate adjuster is used to adjust the flow rate of the fluid or blood flowing inside the medical tube to a suitable rate, and there is a roller clamp in the flow rate adjuster. This roller clamp is provided with respective guide grooves on opposite surfaces of a pair of side wall parts provided on both sides in the longitudinal direction of an elongate plate-like bottom part, and a roller can move in the longitudinal direction of the bottom part in a state in which a shaft part runs between both of said guide grooves. Furthermore, the height between the surface of the bottom part and the guide grooves steadily changes from one end to the other in the longitudinal direction of the bottom part.

Then, with a medical tube placed between the bottom part and the roller, pressure is steadily applied to the medical tube or pressure is steadily released therefrom by changing the position of the roller with respect to the bottom part, whereby the flow rate of the fluid flowing inside the medical tube can be adjusted. However, with this kind of roller clamp, if the medical tube is accidentally pulled by the patient rotating the roller or turning over during sleep, the position of the roller changes, which creates a change in the flow rate of the fluid flowing inside the medical tube. Consequently, a roller clamp cover has been developed with which it is not possible to operate the roller, since the roller clamp is housed inside (see Patent Document 1, for example).

This roller clamp cover (roller clamp covering body) is configured by linking a box body and a lid using a linking part, and an insertion port for the insertion of a medical tube (fluid delivery pipe) is provided on respective opposite surfaces. Furthermore, a latching part for preventing the roller from moving out of its closure position when the roller lies at a position in which the medical tube is closed is provided inside the roller clamp cover. Consequently, if the roller clamp is housed inside the roller clamp cover with the roller lying in a position in which the medical tube is closed, it is not possible for the roller to move out of the closure position, and the medical tube is kept in a closed state.
[Patent Document 1] Japanese Unexamined Utility Model Registration Application H6-48676

### [Disclosure of the Invention]

With the conventional roller clamp described above, the roller clamp is housed inside the roller clamp cover, and therefore there are no opportunities for the patient or another person to rotate the roller and change the position of the roller. Furthermore, the latch part prevents the roller from moving from a position in which the medical tube is closed to a position in which fluid is passing through the medical tube. However, even if the roller clamp is housed inside this kind of roller clamp cover, if the patient turns over during sleep, or people or objects come into contact with the medical tube and the medical tube is suddenly pulled when a drug solution or the like is being supplied to the patient, the position of the roller may change, which causes a change in the flow rate of the fluid flowing inside the medical tube.

The present invention has been devised in view of these matters, and it aims to provide a roller clamp cover which makes it possible to maintain a set flow rate of fluid flowing inside a medical tube to which a roller clamp is fitted.

In order to achieve the aim mentioned above, the structural features of the roller clamp cover according to the present invention lie in the fact that it is a roller clamp cover for housing a roller clamp for adjusting the flow rate of a fluid flowing inside a medical tube by applying pressure using a flow rate adjustment roller to a portion of abovementioned medical tube which is arranged in a tube arrangement part, said roller clamp cover being provided with a housing part comprising a roughly box-shaped member which opens and closes by rotation in reciprocating back and forth directions with a hinge linking part at the centre, said housing part being able to house abovementioned roller clamp in a state in which abovementioned medical tube projects from both sides; and positional offset prevention parts for preventing abovementioned roller clamp from becoming positionally offset from abovementioned medical tube when abovementioned roller clamp is housed inside abovementioned housing part and abovementioned housing part is closed, by applying pressure contact to the outer peripheral surface of abovementioned medical tube.

The roller clamp cover according to the present invention configured in the manner described above is provided with a housing part able to house the roller clamp and positional offset prevention parts which apply pressure contact to the outer peripheral surface of the medical tube when the roller clamp is housed inside the housing part. Accordingly, not only is it possible to prevent operation of the roller part by housing the roller clamp in the housing part, it is also possible to ensure that the positional offset prevention parts come into pressure contact with the outer peripheral surface of the medical tube, so that the position of the roller clamp is not offset from the medical tube. In other words, the positional offset prevention parts come into pressure contact with the outer peripheral surface of the medical tube, whereby, even if the medical tube is pulled by an external force, the positional relationship with the roller clamp does not change, because the medical tube moves together with the housing part, with the positional offset prevention parts in between.

Consequently, it is possible to maintain the flow rate of the fluid flowing inside the medical tube at a set amount even if the medical tube is accidentally pulled. Furthermore, it is possible to use as the roller clamp in this case a device made up of: a tube arrangement part which consists of a bottom part on which the medical tube is arranged from one end to the other in the longitudinal direction, a pair of side wall parts which are provided at both edges in the longitudinal direction of the bottom part, and guide grooves respectively formed on opposite surfaces of the pair of side wall parts and extending as the height between them and the bottom part changes from one end to the other; and also a flow rate adjustment roller which consists of a shaft part of which both ends engage in a mobile fashion with the guide grooves in the pair of side wall parts, and a roller part which is supported by the shaft part.

By means of this, the medical tube which is arranged in the tube arrangement part is pressured onto the bottom part by the roller part by moving the flow rate adjustment roller along both guide grooves while rotating it with the shaft part at the centre so that it is possible to adjust the flow rate of the fluid which is flowing inside the medical tube. Fixing means for fixing the flow rate adjustment roller in a specific position in the tube arrangement part are also more preferably provided on the roller clamp. These fixing means can be configured, among other things, by providing a rod-shaped part or similar on which the medical tube is wound inside the roller clamp to prevent the medical tube from being pulled.

Furthermore, other structural features of the roller clamp cover according to the present invention lie in the fact that the portions on both sides of the housing part where the medical tube projects are configured by a pair of recess parts which are formed on respective matching faces of the roughly box-shaped opening and closing member, and the positional offset prevention parts are configured by resilient members which are attached to the inner surface of the pair of recess parts. In this case, the recess parts are configured by semi-circular recess parts, for example, and they can be formed so as to have a circular hole in them when the housing part is closed. The resilient members consist, for example, of a pair of arc-shaped members made of rubber or sponge, or a synthetic rubber such as silicone rubber, isoprene rubber, butyl rubber or nitrile rubber, or a thermoplastic elastomer. By means of this, it is possible easily to form the positional offset prevention means and the portions of the housing part where the medical tube projects. It should be noted that the roughly box-shaped opening and closing member is a pair of box-shaped bodies or a pair consisting of a box-shaped body and a plate-shaped body which are combined to form substantially a box shape, and the matching faces thereof are in contact when they are closed.

Furthermore, additional structural features of the roller clamp cover according to the present invention lie in the fact that the housing part is formed as a single piece, and the hinge linking part is configured by a thin part which is thinner than the other portions. By means of this, the housing part can be easily formed.

Furthermore, additional structural features of the roller clamp cover according to the present invention lie in the fact that provision is made for fixing means for fixing the flow rate adjustment roller. By means of this, it is possible to stop the roller part more reliably, and it is possible to maintain the flow rate of the fluid flowing inside the medical tube at a set rate. Furthermore, it is possible to use as the fixing means in this case, for example, means whereby the upper part of the flow rate adjustment roller is made to project from the tube arrangement part, and also resilient members are provided in the region of both matching faces of the housing part, so that the upper part of the flow rate adjustment roller is fixedly held between said resilient members. Rubber or sponge, or a synthetic rubber such as silicone rubber, isoprene rubber, butyl rubber or nitrile rubber or thermoplastic elastomer may be used as these resilient members.

### [Optimum Mode of Embodiment of the Invention]

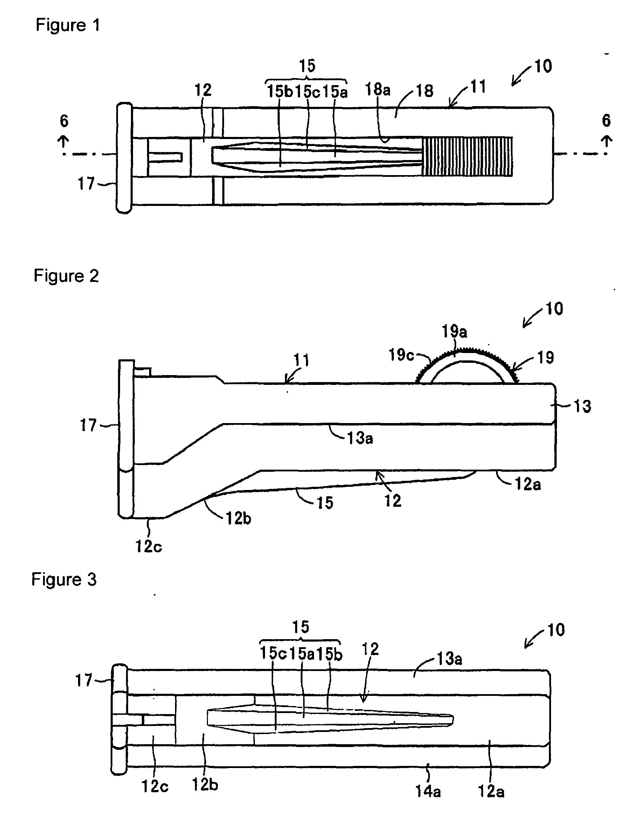

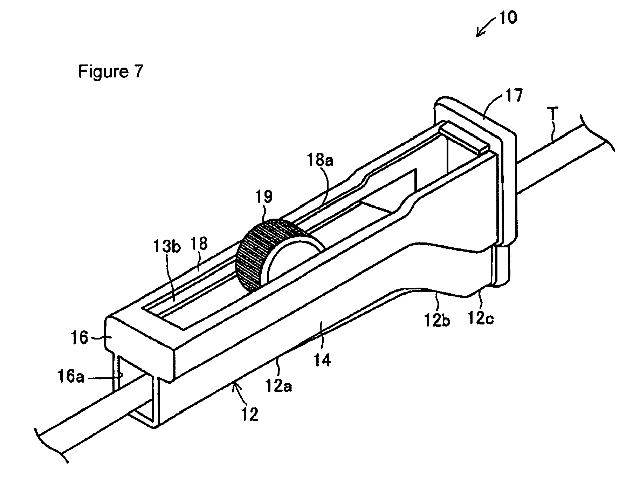

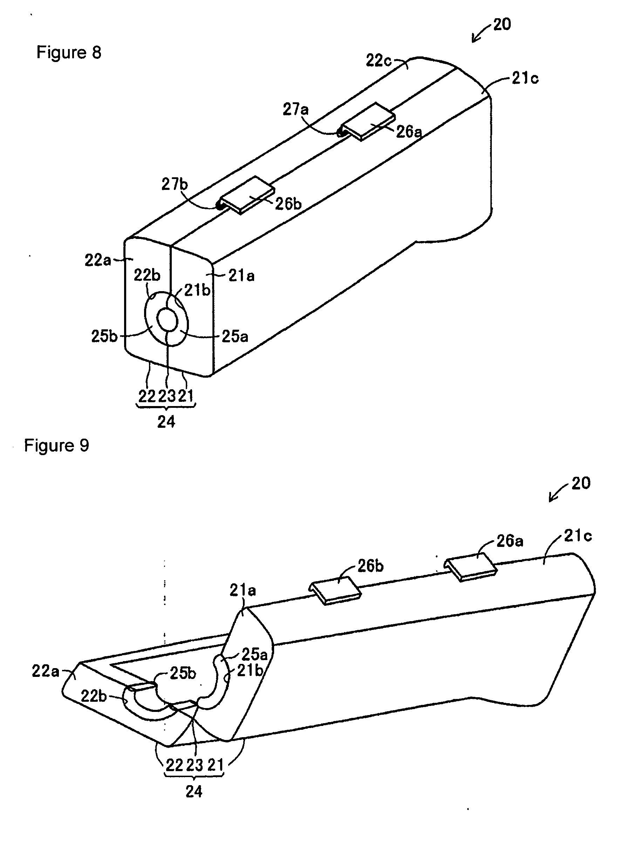

The roller clamp cover according to one mode of embodiment of the present invention will be described in detail below using the figures. Figures 1 to 5 show a roller clamp 10 which is housed inside a roller clamp cover 20 (see Figures 8 and 9) according to this mode of embodiment, and said roller clamp 10 is used in order to adjust the flow rate of a fluid such as a drug solution or blood flowing inside a tube T (see Figure 7) which acts as the medical tube according to the present invention. That is to say, the roller clamp 10 consists of a tube arrangement part 11 which is formed to be roughly trough-shaped, and a flow rate adjustment roller 19 which adjusts the flow rate of the fluid flowing inside the tube T by pressuring the tube T onto the bottom part 12 of the tube arrangement part 11.

The tube arrangement part 11 is formed to be roughly trough-shaped, with side wall parts 13, 14 respectively rising from both edge parts in the longitudinal direction of the elongate plate-shaped bottom part 12. As shown in Figure 3, when the bottom part 12 is seen from the bottom surface, it has a substantially constant width, and it extends in an elongate manner from one end (the front end lying on the right-hand side in Figures 1 to 3; in the description given below, "one end" denotes the right-hand side in Figures 1 to 3, while "the other end" denotes the rear surface on the left-hand side) to the other. Furthermore, as shown in Figure 2, when the bottom part 12 is seen from the side, it consists of a horizontal part 12a which extends horizontally from one end to about 2/3 of the length towards the other end in the longitudinal direction of the bottom part 12, and it consists of an oblique part 12b which is inclined downwards between the other end of said horizontal part 12a and the region of the other end of the bottom part 12.

The portion in the region of the other end of the bottom part 12 is then configured by a short horizontal part 12c. Furthermore, a tube arrangement groove 15 which extends from one end to the other end of the bottom part 12 is formed in the centre of the bottom part 12 in the width direction. Said tube arrangement groove 15 is configured by a groove which is formed with an inner bottom surface 15a which is level and of small width, and it is formed with wall surfaces 15b, 15c comprising oblique facing surfaces the gap between which becomes wider moving upwards from both edge parts which run in the longitudinal direction of said inner bottom surface 15a, so that it is V-shaped in cross section.

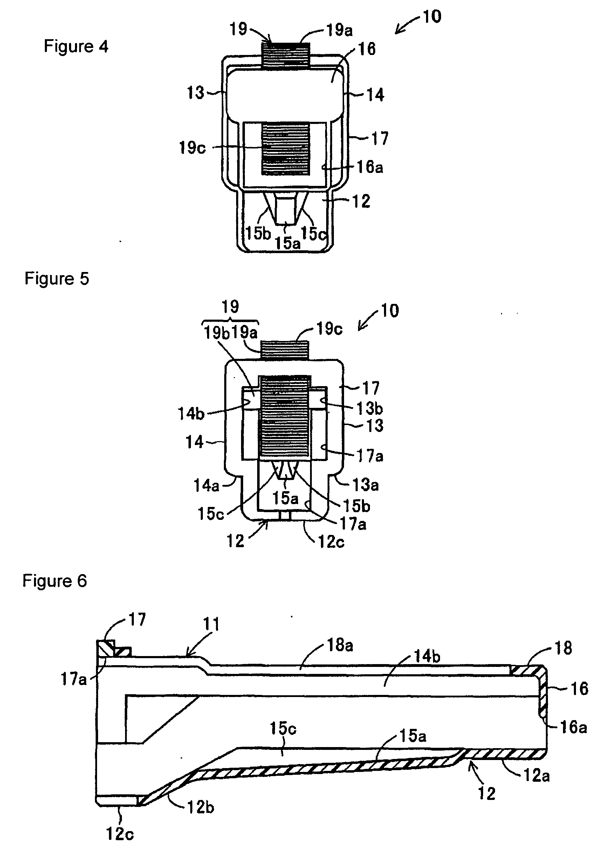

The upper edge parts (the upper surface of the bottom part 12) of the wall surfaces 15b, 15c extend horizontally, as shown in Figure 6, and the inner bottom surface 15a extends obliquely downwards from one end towards the other end. Consequently, the length of the tube arrangement groove 15 in the vertical direction from one end towards the other (the height of the wall surfaces 15b, 15c) becomes gradually greater. The other end of the inner bottom surface 15a then links to the upper surface of the oblique part 12b. Furthermore, the portion at the other end on the upper edge of the wall surfaces 15b, 15c consists of an oblique part which lies on the same face as the upper surface of the oblique part 12b.

The side wall parts 13, 14 bound the bottom part 12 and consist of wall parts which are formed in a laterally symmetrical manner. The lower end edge parts of the side wall parts 13, 14 are formed as curved lines running along the edge parts of the horizontal part 12a, the oblique part 12b and the horizontal part 12c; the upper end edge part of the side wall parts 13, 14 between one end and the portion corresponding to the top of the other end of the tube arrangement groove 15 extends roughly horizontally, with the portion further to the other end than this being formed as a step lying further up than the portion at the one end. Furthermore, step parts 13a, 14a which are parallel with the lower end edge part of the side wall parts 13, 14 are formed roughly in the centre in the vertical direction of the side wall parts 13, 14 and by providing these step parts 13a, 14a, the portion at the upper part of the side wall parts 13, 14 projects further outwards than the portion at the lower part.

The area in-between at one end of the portion at the upper part of the side wall parts 13, 14 is connected by a front wall part 16, as shown in Figure 4, and an insertion opening 16a for the insertion of the tube T is formed in a portion which is surrounded by the lower end edge part of the front wall part 16, a portion at the lower part of the side wall parts 13, 14 at the one end, and the bottom part 12 at the one end. Furthermore, a frame-like part 17 which projects slightly outwards is formed at the other end part of the side wall parts 13, 14, and the area in-between at the other end of the portion at the upper part of the side wall parts 13, 14 is connected by means of the upper part of said frame-like part 17. An insertion opening 17a for the tube T to pass through is formed inside said frame-like part 17.

A frame-like projecting part 18 which projects inwards is formed on the upper end edge part of the side wall parts 13, 14 and the upper end edge part of the front wall part 16, and an elongate guide hole 18a is formed inside said projecting part 18. The inner edge parts of the portions lying along the side wall parts 13, 14 on the projecting part 18 extend as far as a portion corresponding to the inner surface of a portion at the lower part of the side wall parts 13, 14, when seen from above. That is to say, the portion of said tube arrangement part 11 extending from the one end somewhat further to the other end than the central part is formed as a roughly elongate trough shape, the length of which in the height direction is set to be fairly short, and the length in the height direction of the portion at the other end is set to be longer than the height of the portion at the one end, and the bottom part projects downwards, so that the upper part is formed as a short oblique trough shape projecting upwards.

As shown in Figures 5 and 6, guide grooves 13b, 14b are formed on the inner surface facing the side wall parts 13, 14. Said guide grooves 13b, 14b are formed by virtue of the fact that the step parts 13a, 14a are formed on the side wall parts 13, 14, so that the portion at the upper part of the side wall parts 13, 14 projects further outwards than the portion at the lower part, and also the fact that provision is made for the projecting part 18 on the upper end edge part of the side wall parts 13, 14. As described above, the inner edge part of the projecting part 18 extends up to a portion corresponding to the inner surface of the portion at the lower part of the side wall parts 13, 14.

Consequently, opposing parallel faces are formed between the lower surface of the projecting part 18 and the upper face of the step part on the inner surface of the step parts 13a, 14a, and the guide grooves 13b, 14b are formed by said opposing faces and the inner surface of the portion at the upper part of the side wall parts 13, 14. In this way, the inside bottom surface 15a of the tube arrangement groove 15 which is formed on the bottom part 12 runs obliquely downwards from one end to the other end, and the guide grooves 13b, 14b are formed to be horizontal. Consequently, the length between the guide grooves 13b, 14b and the inside bottom surface 15a of the tube arrangement groove 15 grows larger towards the other end, gradually decreasing in size approaching the one end.

The flow rate adjustment roller 19 consists of a disc-shaped roller part 19a of which the thickness in the axial direction is slightly smaller than the width of the guide hole 18a (the space between the portions at the lower part of the side wall parts 13, 14), and of a shaft part 19b which extends through the centre of the roller part 19a and of which both end parts project from the roller part 19a on both sides. A plurality of slide-prevention protrusions 19c are then formed on the peripheral surface of the roller part 19a. The protrusions 19c extend in the axial direction of the roller part 19a, and are formed with a constant spacing around the circumference of the roller part 19a, whereby groove parts are formed over the roller part 19a.

Furthermore, the diameter of the shaft part 19b is set to be slightly smaller than the width in the vertical direction of the guide grooves 13b, 14b. The flow rate adjustment roller 19 which is configured in this manner is inserted into the tube arrangement part 11 in a state in which both end parts of the shaft part 19b are movably positioned inside the guide grooves 13b, 14b. Furthermore, the flow rate adjustment roller 19 is able to move between one end of the tube arrangement part 11 and the other end, in a vertically mobile state over a small width inside the tube arrangement part 11. The roller clamp 10 which is configured in this manner is fitted to the tube T, as shown in Figure 7.

In this case, the tube T is inserted into the insertion opening 17a from the insertion opening 16a, in a state in which the portion at the lower part of the outer peripheral surface is positioned inside the tube arrangement grove 15, and the portion at the upper part of the outer peripheral surface is positioned at the lower surface of the roller part 19a. Consequently, when the flow rate adjustment roller 19 is positioned at the other end of the tube arrangement part 11, the tube T is not pressured by the flow rate adjustment roller 19, and if the flow rate adjustment roller 19 is moved from that position to the one end of the tube arrangement part 11, the tube T is steadily pressured by the flow rate adjustment roller 19 and the flow channel inside grows narrower.

Figures 8 and 9 show the roller clamp cover 20. The roller clamp cover 20 consists of a roughly box-shaped member of a sufficient size to be able to house the roller clamp 10 inside, and it comprises a housing part 24 formed by linking a pair of open/close covers 21, 22 which can cover a respective lateral half of the roller clamp 10 so that said covers can open and close about a hinge linking part 23. The main body portions of the open/close covers 21, 22 are formed to be laterally symmetrical to each other, and, when they are closed, the portion running from one end (the left-hand portion in Figures 8 and 9) to somewhat more towards the other end than the central part is formed to be roughly an elongate box shape which is shorter in the height direction.

The length in the height direction of the portion at the other end when the open/close covers 21, 22 are closed is set to be longer than the length in the height direction of the portion at the one end, and the portion at the other end is formed as a short box shape of which the bottom part protrudes obliquely downwards. The housing part 24 is formed as a single piece, and the hinge linking part 23 is configured by a linking part which is endowed with flexibility by virtue of the fact that it is thin. When the roller clamp 10 in the state shown in Figure 7 is housed at the lower part of the matching faces of front surface parts 21a, 22a of the open/close covers 21, 22, i.e. inside the roller clamp cover 20, semi-circular recess parts 21b, 22b are formed at the portion where the tube T is positioned. Arc-shaped positional offset prevention parts 25a, 25b comprising a resilient material such as rubber are then attached to said recess parts 21b, 22b.

These positional offset prevention parts 25a, 25b form an annular shape when the open/close covers 21, 22 are closed, and the diameter of the hole part which is formed inside at that time is set to be slightly smaller than the outer diameter of the tube T. It is therefore possible to fixedly clamp the tube T using the positional offset prevention parts 25a, 25b. Furthermore, respective semi-circular recess parts are also formed at the lower part of the matching faces at the rear surface part of the open/close covers 21, 22, although these are not depicted, and arc-shaped positional offset prevention parts made of a resilient material such as rubber are attached to the recess parts. That is to say, the tube T projecting from the front and rear of the roller clamp cover 20 is clamped by the positional offset prevention parts 25a, 25b and the positional offset prevention parts provided at the rear surface part, whereby the roller clamp cover 20 can be fixed to the tube T.

Furthermore, a pair of engaging catches 26a, 26b are formed on both sides in the front and rear direction on the matching face side of the upper surface part 21c of the open/close cover 21, and a pair of engaging recess parts 27a, 27b which can engage with the pair of engaging catches 26a, 26b are formed on both sides in the front and rear direction on the matching face side of the upper surface part 22c of the open/close cover 22. The engaging catches 26a, 26b are configured by forming protrusions which extend from front to rear on the tip end lower surface of plate bodies, and the protrusions at the tip end engage by insertion with the engaging recess parts 27a, 27b when the open/close covers 21, 22 are closed, whereby the closed state of the open/close covers 21, 22 is maintained. Furthermore, in this state, it is possible to release the engagement between the engaging catches 26a, 26b and the engaging recess parts 27a, 27b by pressing downwards on the portion close to the engaging recess parts 27a, 27b on the upper surface part 22c.

When the roller clamp 10 and the roller clamp cover 20 configured in this manner are used, the roller clamp 10 is first of all attached to the tube T, then the upstream end of said tube T is connected to a container housing a drug solution or the like, and the downstream end of the tube T is connected to a piercing member such as an indwelling needle which pierces the patient's body and remains indwelling. Next, the drug solution flows into the tube T from the container, and air inside the tube T is expelled to the outside, after which the flow rate adjustment roller 19 is positioned at the one end of the tube arrangement part 11 to stop the flow of drug solution. In this state, the piercing member pierces a specific point on the patient's body, the flow rate adjustment roller 19 is gradually moved to the other end of the tube arrangement part 11, and the flow rate of the drug solution flowing inside the tube T is set to a suitable amount.

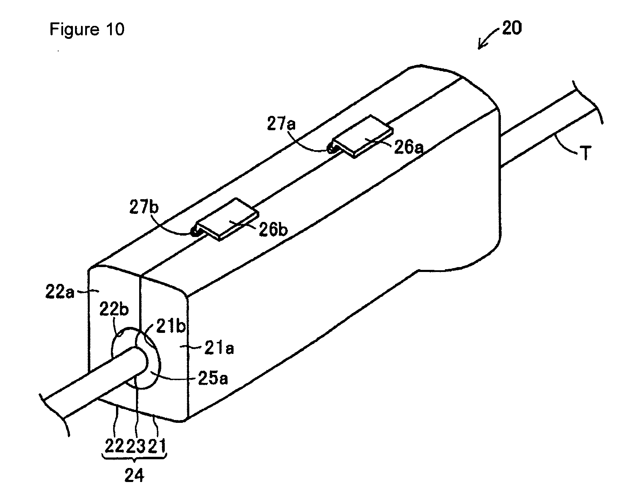

In this case, the flow rate adjustment roller 19 can be maintained in a state in which it is stopped in position, by stopping the rotating action of the flow rate adjustment roller. The roller clamp is then covered by the roller clamp cover 20, to achieve the state shown in Figure 10. This operation is carried out by bringing the hinge linking part 23 side of the roller clamp cover 20 in the state shown in Figure 9 into contact with the lower part of the roller clamp 10, positioning the tube T in the respective positional offset prevention parts 25a, 25b and the positional offset parts provided on the rear surface part, and bringing the open/close covers 21, 22 closer towards each other so as to close them. By means of this, the engaging catches 26a, 26b engage with the engaging recess parts 27a, 27b, and the roller clamp 10 is covered by the roller clamp cover 20.

Furthermore, the portions of the tube T projecting from the front surface parts 21a, 22a and the rear surface parts are clamped by the pressure contact of the positional offset prevention parts 25a, 25b and the positional offset prevention parts provided at the rear surface parts, and the roller clamp cover 20 is in a state in which it is fixed to the tube T. In this case, the flow channel of the tube T at the portion which is gripped by the positional offset prevention parts 25a, 25b etc. is somewhat narrower, but this does affect the flow rate of the drug solution provided that it is wider than the flow channel at the portion gripped by means of the roller clamp 10. By means of this, drug solution at a constant and set flow rate is supplied to the patient's body from the container.

When the drug solution is supplied to the patient, any pulling force on the tube T is transferred to the roller clamp cover 20 and does not reach the roller clamp 10 inside the roller clamp cover 20, even if the patient moves accidentally and pulls on the tube T, or if an object comes into contact with the tube T. Furthermore, when the flow rate of the drug solution flowing inside the tube T is to be changed, the roller clamp cover 20 is removed at one time from the roller clamp 10, and the position of the flow rate adjustment roller 19 is adjusted. Then, after the adjustment, the roller clamp 10 is once again covered by the roller clamp cover 20.

As described above, the roller clamp cover 20 according to this mode of embodiment comprises the housing part 24 for housing the roller clamp 10, and positional offset prevention parts 25a, 25b etc. for applying pressure contact to the outer peripheral surface of the tube T when the roller clamp 10 is housed inside the housing part 24. Accordingly, the roller clamp 10 is housed inside the housing part 24, whereby it is no longer possible to operate the flow rate adjustment roller 19 when a drug solution is being supplied to the patient, and also there is no positional offset between the tube T and the roller clamp 10 even if the tube T is pulled by an external force.

Consequently, it is possible to maintain the flow rate of the fluid flowing inside the tube T at a set amount even if the tube T is accidentally pulled. Furthermore, protrusions 19c are formed on the peripheral surface of the roller part 19a, and therefore the roller part 19a engages suitably with the tube T, and it is possible to prevent the flow rate adjustment roller 19 from becoming positionally offset with respect to the tube T. In addition, the housing part 24 is formed as a single piece, and the hinge linking part 23 is formed by making it thinner than the other portions of the housing part 24, and therefore the housing part 24 can be configured in a simple manner.

Furthermore, in the present invention, resilient members made of rubber or sponge are provided on the inner surface of the upper surface parts 21c, 22c of the open/close covers 21, 22, and when the open/close covers 21, 22 are closed, the portions at the upper part on the side surface of the roller part 19a are gripped from both sides by these resilient members, whereby they can be fixed. By means of this, it is possible to more reliably prevent any positional offset arising between the tube T and the roller clamp 10. In addition, in the present invention, a rod-shaped part for winding of the tube T is provided on the inner part of the roller clamp 10, and the tube T is wound one or more times onto the rod-shaped part, whereby the tube T can be fixed to the roller clamp 10. By means of this, it is possible to more reliably prevent the roller clamp 10 from becoming positionally offset from the tube T.

Furthermore, it is possible to configure the housing part using a box-shaped member and a plate-shaped cover member, or to configure it using a pair of box-shaped members of different widths, and to provide a window part which can open/close on a portion corresponding to the range of movement of the flow rate adjustment roller 19 on the upper surface of the housing part. By means of this, it is possible to open the window part to adjust the position of the flow rate adjustment roller 19, without having to remove the roller clamp cover 20 from the roller clamp 10, when the flow rate of the drug solution flowing inside the tube T is to be changed. The window part which can open/close in this case may be configured by providing a window hole on the main body side of the housing part, and providing a lid which can rotate by way of a hinge linking part on one edge part of said window hole, or by providing a sliding engagement part on the opposite edge part of the window hole and slidably attaching a plate-shaped cover to said sliding engagement part. Furthermore, a detachable engagement part comprising a recess part and a protruding part, for example, is preferably provided between the cover and the main body side of the housing part.

Furthermore, the roller clamp cover according to the present invention is not limited to the mode of embodiment described above, and appropriate modifications may be implemented. For example, in the mode of embodiment described above, the positional offset prevention means 25a, 25b etc. are provided on the matching surfaces on the front surface parts 21a, 22a and the rear surface parts of the open/close covers 21, 22, but it is also possible to form insertion holes for the insertion of the tube T at specific portions at the front surface part and rear surface part of the housing part, and to fix the tube T by pressuring it at specific portions of the open/close covers 21, 22 when the open/close covers 21, 22 are closed. Furthermore, the open/close covers 21, 22 are not limited to laterally symmetrical shapes, and they may be of various shapes.

In addition, in the mode of embodiment described above, the roller clamp 10 and the roller clamp cover 20 are used when a drug solution is supplied to the patient's body, but said roller clamp 10 and roller clamp cover 20 can be used not only for drug solutions, but also when a patient is undergoing a blood transfusion. They can furthermore be used for discharging drainage liquid from a patient's body, or for taking blood, among other things. That is to say, the roller clamp cover according to the present invention can be attached to a roller clamp for use, regardless of the fluid, if there is a fluid passing through a medical tube and a roller clamp is being used.

### [Brief Description of the Figures]

[Figure 1] is a plan view showing the roller clamp which is used in one mode of embodiment of the present invention;
[Figure 2] is a side view of the roller clamp;
[Figure 3] is a bottom view of the roller clamp;
[Figure 4] is a front view of the roller clamp;
[Figure 5] is a rear view of the roller clamp;
[Figure 6] is a view in cross section along 6-6 in Figure 1;
[Figure 7] is an oblique view showing a state in which the roller clamp is attached to the tube;
[Figure 8] is an oblique view showing a state in which the roller clamp cover according to one mode of embodiment of the present invention is closed;
[Figure 9] is an oblique view showing a state in which the roller clamp cover is open; and
[Figure 10] is an oblique view showing a state in which the roller clamp cover is attached to the roller clamp.

### [Explanation of Symbols]

10...roller clamp; 11...tube arrangement part; 12...bottom part; 13, 14...side wall part; 13b, 14b...guide groove; 19...flow rate adjustment roller; 19a...roller part; 19b...shaft part; 19c...protrusion; 20...roller clamp cover; 21, 22...open/close cover; 21b, 22b...recess part; 23...hinge linking part; 24...housing part; 25a, 25b...positional offset prevention part; T...tube.

## Claims

1. Roller clamp cover for housing a roller clamp for adjusting the flow rate of a fluid flowing inside a medical tube by applying pressure using a flow rate adjustment roller to a portion of the abovementioned medical tube which is arranged in a tube arrangement part,
said roller clamp cover being **characterized in that** it is provided with a housing part comprising a roughly box-shaped member which opens and closes by rotation in reciprocating back and forth directions with a hinge linking part at the centre, said housing part being able to house the abovementioned roller clamp in a state in which the abovementioned medical tube projects from both sides; and positional offset prevention parts for preventing the abovementioned roller clamp from becoming positionally offset from the abovementioned medical tube when the abovementioned roller clamp is housed inside the abovementioned housing part and the abovementioned housing part is closed, by applying pressure contact to the outer peripheral surface of the abovementioned medical tube.

2. Roller clamp cover according to Claim 1, in which the portions on both sides of the abovementioned housing part where the abovementioned medical tube projects are configured by a pair of recess parts which are formed on respective matching faces of the abovementioned roughly box-shaped opening and closing member, and the abovementioned positional offset prevention parts are configured by resilient members which are attached to the inner surface of the abovementioned pair of recess parts.

3. Roller clamp cover according to Claim 1 or 2, in which the abovementioned housing part is formed as a single piece, and the abovementioned hinge linking part is configured by a thin part which is thinner than the other portions.

4. Roller clamp cover according to any one of Claims 1 to 3, in which provision is made for fixing means for fixing the abovementioned flow rate adjustment roller.
